# EUROPÄISCHE PATENTSCHRIFT

(11) **EP 1 131 299 B1**
(45) Veröffentlichungstag und Bekanntmachung des Hinweises auf die Patenterteilung: **17.09.2003**
(21) Anmeldenummer: 99972625.0
(22) Anmeldetag: 06.11.1999
(51) Int. Cl.: C07D 231/20

(54) **VERFAHREN ZUR HERSTELLUNG VON 1-SUBSTITUIERTEN 5- ODER 3-HYDROXY-PYRAZOLEN**
METHOD FOR PRODUCING 1-SUBSTITUTED 5- OR 3-HYDROXYPYRAZOLES
PROCEDE DE PREPARATION DE 5- ET/OU 3-HYDROXYPYRALOZES SUBSTITUES EN POSITION 1

(30) Priorität: 19.11.1998 DE 19853502
(43) Veröffentlichungstag der Anmeldung: 12.09.2001
(73) Patentinhaber: BASF AKTIENGESELLSCHAFT, 67056 Ludwigshafen (DE)
(72) Erfinder: MAYWALD, Volker, D-67071 Ludwigshafen (DE); STEINMETZ, Adrian, D-68165 Mannheim (DE); RACK, Michael, D-69123 Heidelberg (DE); GÖTZ, Norbert, D-67547 Worms (DE); GÖTZ, Roland, D-68809 Neulussheim (DE); HENKELMANN, Jochem, D-68165 Mannheim (DE); BECKER, Heike, D-67227 Frankenthal (DE); AISCAR BAYETO, Juan Jose, D-68167 Mannheim (DE)
(86) Internationale Anmeldenummer: EP9908516
(87) Internationale Veröffentlichungsnummer: WO00031042

(56) Entgegenhaltungen:
- EP-A- 0 240 001
- EP-A- 0 587 072
- EP-A- 0 837 058
- US-A- 5 663 365

## Beschreibung

Die vorliegende Erfindung betrifft ein Verfahren zur Herstellung von 1-substituierten 5- und/oder 3-Hydroxy-pyrazolen der Formeln I und II 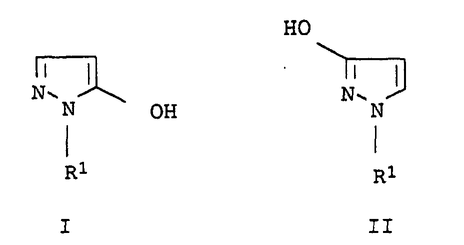 in denen R¹ C₁-C₆-Alkyl, C₂-C₆-Alkenyl, C₂-C₆-Alkinyl, C₃-C₆-Cycloalkyl oder C₁-C₄-Alkoxy bedeutet, wobei diese Gruppen durch Halogen, C₁-C₄-Alkoxy, Phenoxy, C₁-C₆-Alkoxycarbonyl, C₁-C₆-Alkylthiocarbonyl oder ein cyclisches Ringsystem mit 3-14 Ringatomen substituiert sein können.

1-substituierte 5- und 3-Hydroxy-pyrazole finden als Vorprodukte zur Herstellung von Pharmaka und Pflanzenschutzmitteln, insbesondere Herbiziden, Verwendung und sind beispielsweise aus der W096/26206, WO 97/23135, WO 97/19087, US 5,631,210, WO 97/12885, WO 97/08164, ZA 9510980, WO 97/01550, WO 96/31507, WO 96/30368, WO 96/25412 und US 5,663,365 bekannt.

Verfahren zu deren Herstellung sind daher von Interesse.

Bisher sind als Verfahren zur Herstellung von niederen 1-Alkyl-5-hydroxy-pyrazolen folgende Synthesen bekannt:
1. eine Darstellung, bei der 2-Methyl-1-(p-toluolsulfonyl)-3-pyrazolidon oder 2-Methyl-1-acetyl-pyrazolidon hydrolysiert wird (J. Prakt. Chem. 1971, 313, 115-128 und J. Prakt. Chem. 1971, 313, 1118-1124).
2. ein Variante, bei der 5-Hydroxy-1-alkylpyrazol-4-carbonsäurealkylester durch Cyclisierung eines Alkoxymethylenmalonsäuredialkylesters mit niederen Alkylhydrazinen aufgebaut wird, anschließend zu diesem Reaktionsprodukt eine wäßrige Mineral-Säure-Lösung zugesetzt, und die Hydrolyse und Decarboxylierung gleichzeitig durchgeführt wird (siehe JP 61257974, JP 60051175, JP 58174369, JP 58140073 und JP 58140074 sowie US 4643757).
3. eine Synthese, bei der Propiolsäureethylester mit Methylhydrazin zu 5-Hydroxy-1-methyl-pyrazol umgesetzt wird (Annalen 1965, 686, 134-144).
4. eine Syntheseroute, bei der 3-Hydrazinopropionsäureester, die durch Addition von Hydrazin an Acrylsäureester entstehen, mit Aldehyden zu den entsprechenden Hydrazonen umgesetzt und anschließend cyclisiert werden (siehe JP 06166666, JP 61229852 und JP 61268659 sowie EP 240001).
5. ein Synthesevariante, bei der eine 5-Hydroxy-1-methylpyrazol-3-carbonsäure thermisch gespalten wird (Chem. Ber. 1976 109, S. 261).
6. ein Verfahren, bei dem 3-Alkoxyacrylsäureester mit Methylhydrazin bzw. Ethylhydrazin zu 1-Methyl-5-hydroxy-pyrazol bzw. 1-Ethyl-5-hydroxy-pyrazol umgesetzt werden (siehe JP 189 271/86, EP-A-837 058).
7. ein Verfahren, bei dem 2-Haloacrylsäureester mit Hydrazinderivaten zu 1- substituierten 3-Hydroxypyrazolen umgesetzt werden (siehe US 5,663,365).

Bei dem oben genannten 1. Syntheseweg ist der Verfahrensprozeß mehrstufig und kompliziert. Das Einfügen und Entfernen einer Schutzgruppe ist umständlich, bedeutet zusätzliche Stufenzahlen und vermindert die Ausbeute.

Die 2. Darstellungsmöglichkeit ist mehrstufig, außerdem entstehen neben den 1-Alkyl-5-hydroxy-pyrazolen gleichzeitig die Regioisomeren der Zielverbindung, die aufwendig von den Zielverbindungen abgetrennt werden müssen. Zudem ist die Synthese mit einer schlechten C-Ausbeute verbunden, da ein C4-Baustein eingesetzt wird, wovon am Ende des Verfahrens wieder ein C-Atom abgespalten werden muß.

Bei der 3. Synthesevariante, die lediglich die Darstellung von 1-Methyl-5-hydroxy-pyrazol beschreibt, ist es unabdingbar, weit überstöchiometrische Mengen an Methylhydrazin einzusetzen, wodurch das Verfahren unwirtschaftlich ist. Daneben muß das ebenfalls entstehende Isomer 3-Hydroxy-1-methylpyrazol bei der Reinigung aufwendig von 1-Methyl-5-hydroxy-pyrazol getrennt werden.

Des weiteren ist dieses Verfahren aufgrund des hohen Preises von Propiolsäureester unwirtschaftlich.

Bei der 4. Alternative ist der Verfahrensprozeß mehrstufig und kompliziert. Die letzte Stufe des aufwendigen Verfahrens liefert nur schlechte Ausbeuten und eine Vielzahl von Nebenprodukten.

Bei dem 5. Synthesezugang ist zur thermischen Abspaltung eine hohe Temperatur erforderlich, die Ausbeute ist mit 6% sehr gering.

Beim 6. Syntheseweg, der lediglich die Darstellung von 1-Methyl-5-hydroxy-pyrazol beschreibt, werden aufwendig darzustellende und teure 3-Alkoxyacrylsäureester eingesetzt. Die Herstellung von 3-Alkoxyacrylsäureester erfolgt durch Umsetzung von Methanol mit teuren Propiolsäurestern (Tetrahedron Lett. 1983, 24, 5209, J. Org. Chem. 1980, 45, 48, Chem. Ber. 1966, 99, 450, Chem. Lett. 1996, 9, 727-728), durch Umsetzung teurer und aufwendig zu synthetisierenden α,α-Dichlor-diethylether mit Bromessigsäureestern (Zh. Org. Khim. 1986, 22, 738), durch Umsetzung von Bromessigsäureestern mit Trialkylformiaten (Bull. Soc. Chim. France 1983, N 1-2, 41-45) und durch Abspaltung von Methanol aus 3,3-Dialkoxypropionsäureestern (DE 3701113) (erhältlich durch Umsetzung des teuren Propiolsäuremethylester mit Methanol (J. Org. Chem. 1976, 41, 3765), durch Umsetzung von 3-N-Acetyl-N-alkyl-3-methoxy-propionsäureestern mit Methanol (J. Org. Chem. 1985, 50, 4157-4160, JP 60-156643), durch Umsetzung von Acrylsäureestern mit Alkylaminen und Essigsäureanhydrid (J. Org. Chem. 1985, 50, 4157-4160), durch Umsetzung von Keten mit Trialkylorthoformiat (DK 158462), durch Palladium-und gleichzeitiger Kupfer-katalysierte Reaktion von Acrylsäureestern mit Methanol (DE 4100178.8), durch Umsetzung von Trichloracetylchlorid mit Vinylethylether (Synthesis 1988, 4, 274), durch Umsetzung von α,α,α-Trichlor-ß-methoxy-buten-2-on mit Methanol (Synthesis 1988,4, 274) und durch Umsetzung der Natriumsalze von 3-Hydroxyacrylsäureestern mit Alkoholen (DB 3641605) eingesetzt werden. Die schlechte Zugänglichkeit der 3-Alkoxyacrylsäureester macht die Synthese nach 6. daher unwirtschaftlich. Nach JP 189 271/86 wird außerdem lediglich die Isolierung des 5-Hydroxy-1-methylpyrazols als Hydrochlorid beschrieben, jedoch keine Aussage zur Isolierung und Reinigung der freien Base beschrieben. Versucht man die in JP 189 271/86 beschriebenen Reaktionsbedingungen anzuwenden und die freie Base zu isolieren, erhält man nur sehr geringe Ausbeuten, die für eine großtechnische Herstellung von Hydroxypyrazolen unwirtschaftlich sind.

Der 7. Synteseweg hat den Nachteil, daß ausschließlich 3-Hydroxypyrazole herstellbar sind und keine 5-Hydroxypyrazole.

Folglich können diese Syntheserouten als wirtschaftliche und effiziente Verfahren zur Herstellung von 1-substituierten 5- und 3-Hydroxypyrazolen nicht zufriedenstellen.

Weiterhin ist kein Verfahren aus dem Stand der Technik bekannt, das es erlaubt, durch einfache Variation der Verfahrensparameter sowohl das 1-substituierte 5- als auch das 3-Hydroxypyrazol herzustellen.

Darüberhinaus ist kein Verfahren aus dem Stand der Technik bekannt, das von einfachen Ausgangsstoffen wie einem Alkylvinylether zu den gewünschten 1-substituierten 5-und 3-Hydroxypyrazolen führt.

Aufgabe der vorliegenden Erfindung war es daher, ein Verfahren zu finden, mit dem man in durch Änderung der Verfahrnesparameter 1-substituiert 5-Hydroxypyrazole und/oder 3-Hydroxypyrazole herstellen kann.

Weitere Aufgabe der vorliegenden Erfindung war es, ein Verfahren zur Herstellung von 1-substituierten 5-Hydroxypyrazolen und/oder 3-Hydroxypyrazolen aus leicht zugänglichen Ausgangskomponenten zu finden, das die oben genannten Nachteile der bisher bekannten Verfahren nicht aufweist.

Diese Aufgabe wurde überraschenderweise gelöst durch das erfindungsgemäße Verfahren zur Herstellung von 1-substituierten 5- und/oder 3-Hydroxypyrazolen der Formeln I und II, 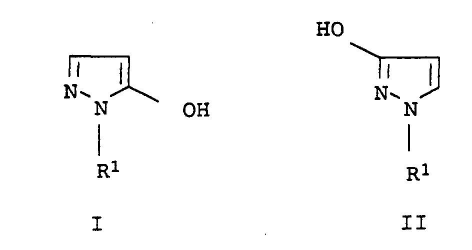 in denen R¹ C₁-C₆-Alkyl, C₂-C₆-Alkenyl, C₂-C₆-Alkinyl, C₃-C₆-Cycloalkyl, C₁-C₄-Alkoxy oder Phenoxy bedeutet, wobei diese Gruppen durch Halogen, C₁-C₄-Alkoxy, C₁-C₆-Alkoxy-carbonyl, C₁-C₆-Alkylthiocarbonyl oder ein cyclisches Ringsystem mit 3-14 Ringatomen substituiert sein kann, indem man
a) einen Alkylvinylether der allgemeinen Formel V, 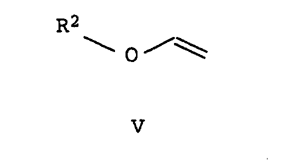 in der R² die in Anspruch 1 angegebene Bedeutung hat, mit Phosgen VIa, "Diphosgen" VIb oder "Triphosgen" VIc 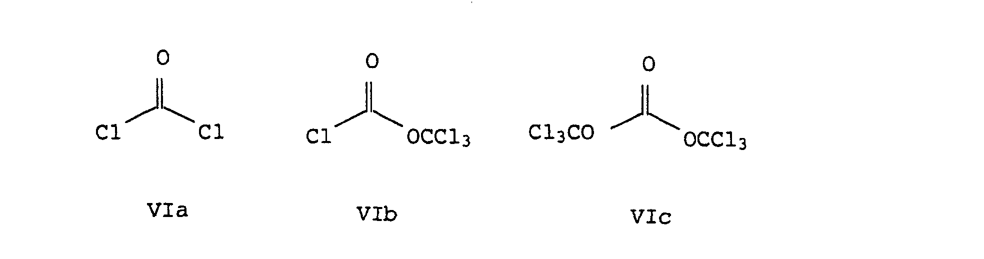 zu Säurechloriden der Formel VII 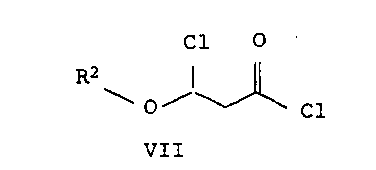 umsetzt,
b) diese durch Eliminierung von Chlorwasserstoff in das entsprechende 3-Alkoxyacrylsäurechlorid der Formel VIII 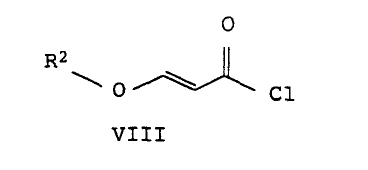 überführt und
c) dieses mit einem Alkohol der Formel IX 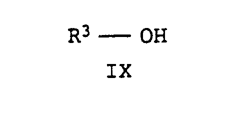 in der R³ die in Anspruch 1 angegebene Bedeutung hat, zu dem entsprechenden 3-Alkoxyacrylsäurealkylester der Formel III verestert.
diesen 3-Alkoxyacrylsäurealkylester der Formel III, 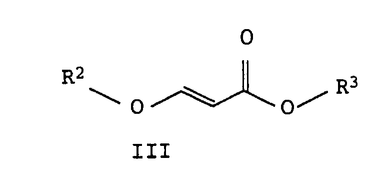 in der R^{2,} R³ unabhängig voneinander C₁-C₆-Alkyl oder C₃-C₆-Cycloalkyl bedeutet, mit einem Hydrazin der Formel IV 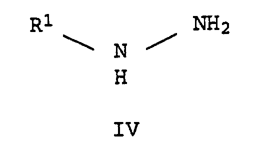 in der R¹ die oben angegebene Bedeutung hat,
d) bei einem pH-Wert von 6-11 zu 5-Hydroxypyrazolen der Formel I umsetzt,
   oder
e) bei einem pH-Wert von 11-14 zu 3-Hydroxypyrazolen der Formel II umsetzt.

Überraschend und neu beim erfindungsgemäßen Verfahren ist, daß durch geeignete Wahl der Reaktionsbedingungen wahlweise 5- oder 3-Hydroxypyrazole der Formeln I bzw. II hergestellt werden können und daß von einfach zugänglichen Ausgangsverbindungen ausgegangen werden kann.

Bevorzugte Ausführungsformen des erfindungsgemäßen Verfahrens sind den Unteransprüchen und der nachfolgenden Beschreibung zu entnehmen.

### stufe d) :

Bei der Umsetzung der 3-Alkoxyacrylsäurealkylester der Formel III mit Hydrazinen der Formel IV zu den 1-substituierten 5-Hydroxypyrazolen geht man im allgemeinen so vor, daß man einen der beiden Reaktanden in einem geeigneten Lösungsmittel vorlegt und den zweiten Reaktanden bei Temperaturen zwischen -30°C und 100°C zudosiert. Der pH-Wert wird durch Zugabe einer Base auf 7 - 11, bevorzugt 8 - 11, besonders bevorzugt 9 - 11 gehalten. Geeignete Basen sind beispielsweise Alkali- und Erdalkalihydroxide wie Natrium- und Kaliumhydroxid sowie tert. Amine.

Bevorzugt werden Alkali- und Erdalkalihydroxide wie Natrium- und Kaliumhydroxide als Base verwendet. Das Molverhältnis von 3-Alkoxyacrylsäurealkylester III zu Hydrazinen IV beträgt 1:1 bis 1:10, bevorzugt 1:1 bis 1:8. Dieses Verhältnis kann man durch Zugabe von Basen von 1:10 bis auf 1:1 verringern.

Nach einer bevorzugten Vorgehensweise legt man nur das Lösungsmittel vor und tropft das Hydrazin IV und den 3-Alkoxyacrylsäurealkylester III gleichzeitig innerhalb 10 min bis 10 h, vorzugsweise 1-4 h zu. Der besondere Vorteil dieser Paralleldosierung liegt darin, daß hierdurch der pH-Wert des Reaktionsgemisches ohne die Notwendigkeit der Zugabe einer Base konstant bei ca.10 gehalten werden kann. Die Einhaltung dieses pH-Werts wiederum ist essentiell für die Regioselektivität der Reaktion. Beispielsweise können bei Einhaltung des pH-Werts auf 10 Regioisomerenverhältnisse I : II von über 300 : 1 erzielt werden.

Als günstig erwiesen hat sich außerdem, die Temperatur nach einer gewissen Reaktionszeit abzusenken und die Reaktion bei entsprechend niedrigerer Temperatur zu vervollständigen.

Geeignete Lösungs- oder Verdünnungsmittel sind beispielsweise Wasser, aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Alkohole wie Methanol und Ethanol sowie Ether wie Diethylether, Diisopropylether, tert.-Butylmethylether, Dioxan, Anisol und Tetrahydrofuran, und Nitrile wie Acetonitril und Propionitril. Natürlich können auch Gemische der genannten Lösungsmittel verwendet werden.

Bevorzugte Lösungsmittel sind beispielsweise Wasser, Alkohole wie Methanol und Ethanol, Ether wie Diethylether, Diisopropylether, tert. Butylmethylether, Dioxan, Anisol, Diethylenglycoldialkylether und Tetrahydrofuran sowie deren Gemische.

Die Hydrazine IV können sowohl in Substanz als auch in Form ihrer z.T. kommerziell erhältlichen wäßrigen Lösungen eingesetzt werden.

### Stufe e) :

Bei der Umsetzung der 3-Alkoxyacrylsäurealkylester III mit Hydrazinen IV zu den 1-substituierten 3-Hydroxy-pyrazolen II geht man vorzugsweise so vor, daß man das Hydrazin IV in einem geeigneten Lösungsmittel vorlegt und bei Temperaturen von -30°C bis 100°C, bevorzugt bei 10-40°C, innerhalb von 10 min bis 10 h vorzugsweise 1-4 h den 3-Alkoxyacrylsäurealkylester VIII zudosiert. Der pH-Wert wird hierbei durch Zugabe einer Base zwischen 11 und 14, vorzugsweise 12-13, insbesondere 12 gehalten. Durch Einstellung des pH-Werts auf die zuletzt genannten Werte gelingt es, die 1-substituierten 3-Hydroxy-pyrazole II in hoher Regioselektivität zu erhalten. Geeignete Basen sind Alkali- und Endalkalimetallhydroxide wie Natrium- und Kaliumhydroxid und tert. Amine. Bevorzugte Basen swind Alkali- und Erdalkalimetallhydroxide. Geeignete Lösungsmittel sind die in Stufe a) genannten.

### Stufe a) :

Ausgangspunkt des erfindungsgemäßen Gesamt-Verfahrens sind Alkylvinylether der Formel V, die zunächst bei Temperaturen von -78°C bis 100°C, vorzugsweise -10°C bis 80°C, insbesondere 20°C bis 60°C, mit einem Säurechlorid der Formel VIa, VIb oder VIc umgesetzt werden, wobei sich das entsprechende Säurechlorid der Formel VII bildet.

Die Umsetzung kann ohne Verwendung von Lösungs- bzw. Verdünnungsmitteln durchgeführt werden, sofern die Umsetzungspartner bei der Umsetzungstemperatur flüssig sind. Es ist aber auch möglich, die Umsetzung in einem aprotischen Lösungs- oder Verdünnungsmittel durchzuführen.

Geeignete Lösungs- oder Verdünnungsmittel sind beispielsweise aliphatische Kohlenwasserstoffe wie Pentan, Hexan, Cyclohexan und Petrolether, aromatische Kohlenwasserstoffe wie Toluol, o-, mund p-Xylol, halogenierte Kohlenwasserstoffe wie Methylenchlorid, Chloroform und Chlorbenzol, sowie Ether wie Diethylether, Diisopropylether, tert.-Butylmethylether, Dioxan, Anisol und Tetrahydrofuran, und Nitrile wie Acetonitril und Propionitril. Natürlich können auch Gemische der genannten Lösungsmittel verwendet werden.

Besonders bevorzugt führt man die Umsetzung ohne Lösungsmittel oder in aromatischen Kohlenwasserstoffen wie Toluol als Lösungsmittel durch.

Die Umsetzungspartner V und VI werden im allgemeinen im Verhältnis von 0,1:1 bis 1:1 mol V/VIa, VIb bzw. VIc, vorzugsweise 0,2:1 bis 0,8:1 mol V/VIa, VIb bzw. VIc, insbesondere 0,4:1 bis 0,6:1 mol V/VIa, VIb bzw. VIc, miteinander umgesetzt.

Da sowohl die Halogenide VI als auch das gebildete Säurechlorid VII gegen Feuchtigkeit labil sind, empfiehlt es sich, die Umsetzung unter Ausschluß von Wasser, vorzugsweise unter Schutzgasatmosphäre (Stickstoff oder ein anderes Inertgas) durchzuführen.

Im Fall der Umsetzung von V mit VIb bzw. VIc kann es von Vorteil sein, die Reaktion durch die Zugabe von katalytischen Mengen eines tertiären Amins wie Triethylamin und Pyridin zu beschleunigen.

### Stufe b):

Das so gewonnene Säurechlorid VII spaltet bei 30 bis 80°C Chlorwasserstoff (HC1) ab, wobei sich das entsprechende 3-Alkoxyacrylsäurechlorid VIII bildet.

Für diese Stufe der Umsetzung kann es von Vorteil sein, den gebildeten Chlorwasserstoff aus dem Reaktionsvolumen zu entfernen, sei es durch leichten Unterdruck oder dadurch, daß man Inertgas durch die Reaktionsmischung oder das Reaktionsgefäß leitet und somit den gebildeten Chlorwasserstoff entfernt.

Das überschüssige Chlorid der Formel VIa, VIb bzw. VIc kann in die Synthese rückgeführt werden und muß in jedem Fall zur Isolierung des reinen Wertproduktes abgetrennt werden. Gleiches gilt für evtl. zugesetzte Katalysatoren.

Die so erhaltenen rohen 3-Alkoxyacrylsäurechloride VIII können durch Destillation oder Rektifikation in reiner Form isoliert werden.

Sie können aber auch direkt, ohne weitere Reinigung, in die entsprechenden 3-Alkoxyacrylsäurealkylester III überführt werden.

### Stufe c):

Bei der Veresterung der Säurechloride VIII geht man im allgemeinen so vor, daß man zum Säurechlorid VIII den Alkohol IX bei Temperaturen von -20 bis 80°C, vorzugsweise 0 - 50°C innerhalb von 0,5 - 8 h vorzugsweise 1-6 h zutropft und den enthaltenen 3-Alkoxyacrylsäurealkylester III durch kontinuierliche oder diskontinuierliche Destillation oder Rektifikation reinigt.

Es ist aber auch möglich, die Umsetzung in einem aprotischen Lösungs- oder Verdünnungsmittel durchzuführen. Geeignete Lösungsoder Verdünnungsmittel sind beispielsweise aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, sowie Ether wie Diethylether, Diisopropylether, tert.-Butylmethylether, Dioxan, Anisol und Tetrahydrofuran, und Nitrile wie Acetonitril und Propionitril. Natürlich können auch Gemische der genannten Lösungsmittel verwendet werden.

Es empfiehlt sich, die Reaktion in Anwesenheit chlorwasserstoffbindender Reagenzien wie z.B. Pyridin durchzuführen. Selbstverständlich können die zuletzt genannten Reagenzien auch als Lösungsmittel verwendet werden.

Im Hinblick auf die bestimmungsgemäße Verwendung der 1-substituierten 5- und/oder 3-Hydroxypyrazolen der allgemeinen Formeln I und II kommen als Substituenten folgende Reste in Betracht:
R¹
   C₁-C₄-Alkyl, wie Methyl, Ethyl, n-Propyl, 1-Methylethyl, Butyl, 1-Methylpropyl, 2-Methylpropyl und 1,1-Dimethylethyl;
   C₁-C₆-Alkyl, wie C₁-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
   insbesondere Methyl, Ethyl, 1-Methylethyl, 1-Methylpropyl, 2-Methylpropyl, 1,1-Dimethylethyl und 1,1-Dimethylpropyl; C₂-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 1-Methyl-3-butenyl, 2-Methyl-4-butenyl, 3-Methyl-3 butenyl, 1,1-Dimethyl-2-propenyl,1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3 pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und Ethyl-2-methyl-2-propenyl,
   insbesondere 1-Methyl-2-propenyl, 1-Methyl-2-butenyl, 1,1-Dimethyl-2-propenyl und 1,1-Dimethyl-2-butenyl;
   C₂-C₆-Alkinyl wie Propargyl, 2-Butinyl, 3-Butinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
   C₃-C₆-Cycloalkyl, wie beispielsweise Cyclopropyl, Cyclobutyl, Cyclopentyl und Cyclohexyl,
   insbesondere Cyclopropyl und Cyclohexyl;
   C₁-C₄-Alkoxy wie Methoxy, Ethoxy, n-Propoxy, 1-Methylethoxy, n-Butoxy, 1-Methylpropoxy, 2-Methylpropoxy und 1,1-Dimethylethoxy,
   insbesondere C₁-C₃-Alkoxy wie Methoxy, Ethoxy, i-Propoxy;
   wobei diese Gruppen gegebenenfalls durch ein bis fünf Halogenatome wie Fluor, Chlor, Brom und Iod, vorzugsweise Fluor und Chlor, C₁-C₄-Alkoxy, Phenoxy, C₁-C₆-Alkoxycarbonyl, C₁-C₆-Alkylthiocarbonyl oder ein cyclisches Ringsystem mit 3-14 Ringatomen substituiert sein können, wobei die Substituenten die folgende Bedeutung haben:
   C₁-C₆-Alkoxycarbonyl wie Methoxycarbonyl, Ethoxycarbonyl, n-Propoxycarbonyl, 1-Methylethoxycarbonyl, n-Butoxycarbonyl, 1-Methylpropoxycarbonyl, 2-Methylpropoxycarbonyl und 1,1-Dimethylethoxycarbonyl, insbesondere Methoxycarbonyl;
   C₁-C₆-Alkylthiocarbonyl wie Methylthiocarbonyl, Ethylthiocarbonyl n-Propylthiocarbonyl, insbesondere Methylthiocarbonyl.
   C₁-C₄-Halogenalkyl: einen C₁-C₄-Alkylrest wie vorstehend genannt, der partiell oder vollständig durch Fluor, Chlor, Brom und/oder Iod substituiert ist, also z. B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;

Ein cyclisches Ringsystem mit 3 - 14 Ringatomen bedeutet beispielsweise die folgenden Gruppen: C₃-C₁₄-Cycloalkyl, C₃-C₁₄-Cycloalkenyl, aromatische Gruppen, wie Phenyl, Naphthyl, sowie deren teilhydrierten Derivate. Ferner können die cyclischen Ringsysteme heterocyclische Ringsysteme darstellen, in denen ein, zwei oder drei Kohlenstoffatome durch Heteroatome, wie z.B. O, N, S, ersetzt sein können. Grundsätzlich können die cyclischen Ringsysteme aromatisch, teilhydriert oder vollständig hydriert sein. Die cyclischen Ringsysteme können beliebig substituiert sein. Als Substituenten geeignet sind beispielsweise C₁-C₆-Alkyl, C₁-C₄-Halogenalkyl, C₁-C₄-Alkoxy, Halogen, Cyano, Nitro, Hydroxyl, Thionyl, Sulfoxyl, Sulfonyl, C₁-C₄-Alkylsulfonyl, Amino, C₁-C₄-Alkylamino und Di-C₁-C₄-Alkylamino.

Bevorzugt sind cyclische Ringsysteme aus der Gruppe C₁-C₆-Cycloalkyl, Phenyl, ein 5 bis 6-gliedriger heterocyclischer, gesättigter oder ungesättigter Rest, enthaltend ein bis drei Heteroatome, ausgewählt aus der Grupppe O, N und S, die wie oben angegeben substituiert sein können.

Besonders bevorzugt ist C₁-C₆-Cycloalkyl und Phenyl, die wie oben angegeben substituiert sein können.

Ein ganz besonders bevorzugtes cyclisches Ringsystem ist Phenyl, das wie oben angegeben substituiert sein kann.

R², R³ unabhängig voneinander C₁-C₆-Alkyl wie oben genannt oder C₃-C₆-Cycloalkyl, bevorzugt C₁-C₆-Alkyl.

### Beispiele

### Beispiel 1

### 3-Ethoxyacrylsäurechlorid

Zu einer Lösung aus 72 g (1 mol) Ethylvinylether in 100 g Toluol werden 110 g (1,1 mol) Phosgen bei 35°C innerhalb von 1,5 h eingeleitet. Anschließend wird 4 Stunden bei 60°C nachgerührt. Während der gesamten Versuchszeit werden Phosgen und Ethylvinylether mit einem -78°C-Trockeneiskühler in die Reaktionsmischung rückkondensiert. Anschließend wird die Lösung bei Raumtemperatur phosgenfrei gestrippt und das Lösungsmittel destillativ entfernt. Nach Vakuumdestillation bei 36°C/0,4 mbar werden 88 g (66 %) Wertprodukt erhalten.

### Beispiel 2

### 3-Isobutoxyacrylsäurechlorid

100 g (1 mol) Isobutylvinylether werden in einer 2 1 Rührapparatur vorgelegt und auf 50-55°C erwärmt. Anschließend werden 1024 g (10,4 mol) Phosgen innerhalb von 21 h eingeleitet und 900 g (9 mol) Isobutylvinylether innerhalb von 19 h zugetropft. Nach einer Nachreaktionszeit von 0,5 Stunden wird der Ansatz unter Stickstoffstrippung auf 80°C erwärmt, um Chlorwasserstoff abzuspalten. Danach werden die Niedersieder über eine 15 cm Vigreux-Kolonne abdestilliert und der Rückstand gaschromatographisch analysiert. Es werden 1551 g (80%) rohes Isobutoxyacrylsäurechlorid (ber. 100%) erhalten.

### Beispiel 3

### 3-Cyclohexyloxyacrylsäurechlorid

In eine mit -78°C-Kühlung ausgestattete Rührapparatur werden 50 g (0,5 mol) Phosgen einkondensiert. Anschließend werden innerhalb von 3 Stunden 50,5 g (0,4 mol) Cyclohexylvinylether bei 20°C eingetropft. Danach wird 5 Stunden bei 50°C nachgerührt. Das überschüssige Phosgen wird mit Stickstoff ausgetrieben und das Rohprodukt destillativ aufgearbeitet. Bei 110°C/2,5mbar wurden 66,4g (88 %) Wertprodukt erhalten.

### Beispiel 4

### Isobutoxyacrylsäureisobutylester

In einer 500 ml Rührapparatur werden 100 g (1,0 mol) Isobutylvinylether vorgelegt und auf 50-55°C erwärmt. Anschließend werden 113 g (1,15 mol) Phosgen über 11 h eingeleitet. Nach einer Nachreaktionszeit von 1,5 h wird das Reaktionsgemisch bei 50-55°C durch Einleiten von Stickstoff phosgenfrei gestrippt. Anschließend läßt man auf Raumtemperatur abkühlen und und tropft 60,7 g (0,82 mol) iso-Butanol zu. Nach vollendeter Zugabe wird das erhaltene Reaktionsgemisch rektifiziert. Man erhält 150,4 g (75%) 3-Isobutoxyacrylsäureisobutylester vom Sdp. 97°C bei 2,5 mbar.

### Beispiel 5

### 5-Hydroxy-1-methylpyrazol aus 3-Methoxyacrylsäuremethylester und Monomethylhydrazin (35%)

In einem 250 ml Kolben werden zu 70 g Methanol bei 25 °C 106,2 g (0,808 mol) 35%iges wäßriges Monomethylhydrazin und 47,82 g (0,404 mol) 3-Methoxyacrylsäuremethylester gleichzeitig innerhalb von 25 min zudosiert. Nach 2 Stunden Rühren bei 25 °C wird das Reaktionsgemisch gaschromatographisch analysiert. Die Ausbeute beträgt 95 % bei 100 % Umsatz (jeweils bezogen auf 3-Methoxyacrylsäuremethylester).
Isomerenverhältnis: (5-Hydroxyisomer: 3-Hydroxyisomer) ≥ 200 :1

### Beispiel 6

### 5-Hydroxy-1-methylpyrazol aus 3-Isobutoxyacrylsäureisobutylester und Monomethylhydrazin (35 %)

In einem 0.75 1 Reaktor werden zu 202 g Methanol bei 25 °C 287,5 g (2,18 mol) 35%iges wäßriges Monomethylhydrazin und 175,2 g (0,875 mol) 3-Isobutoxyacrylsäure-isobutylester gleichzeitig innerhalb von 1,5 Stunden zudosiert. Nach 6,75 Stunden Rühren bei 25 °C wird das Reaktionsgemisch für 16 Stunden auf 5 °C abgekühlt und anschließend gaschromatographisch analysiert. Die Ausbeute beträgt 88 % bei 98 % Umsatz (jeweils bezogen auf Isobutoxyacrylsäureisobutylester)
Isomerenverhältnis: (5-Hydroxyisomer: 3-Hydroxyisomer) ≥ 300 :1)

### Beispiel 7

### 3-Hydroxy-1-methylpyrazol aus 3-Methoxyacrylsäuremethylester und Monomethylhydrazin (35%)

In einem 250 ml Kolben werden 70 g Methanol und 60,5 g (0,46 mol) 35%iges wäßriges Monomethylhydrazin bei 25 °C vorgelegt. Innerhalb von 25 min werden bei gleicher Temperatur 47,8 g (0,40 mol) 3-Methoxyacrylsäuremethylester zudosiert. Durch parallele Zugabe von 17 %iger wässriger NaOH-Lösung wird der pH-Wert der Reaktionsmischung während des Zudosierens des Methöxyacrylsäuremethylesters und während der Nachrührzeit (6 Stunden) auf 12 eingestellt. Es werden in diesem Zeitraum 97,5 g NaOH-Lösung verbraucht. Die Ausbeute beträgt 75% bei 100% Umsatz. Isomerenverhältnis: (3-Hydroxyisomer: 5-Hydroxyisomer) ≥ 15 : 1

### Beispiel 8

### 1-Ethoxycarbonylmethyl-5-hydroxypyrazol aus 3-Methoxyacrylsäuremethylester und Hydrazinessigsäureethylester-Hydrochlorid

In einem 2 1 Rundkolben werden bei 25°C 85,5 g (0,55 mol) Hydrazinessigsäureethylester-Hydrochlorid in 770 ml Ethanol vorgelegt. Innerhalb von 1,25 Stunden werden bei 60-65°C 63,8 g (0,55 mol) 3-Methoxyacrylsäuremethylester zudosiert. Nach beendeter Zugabe wird 2 Stunden unter Rückfluß erhitzt und anschließend mit 30%iger methanol.Na-methylat-Lösung auf einen pH-Wert von 5 gestellt. Anschließend wird das Reaktionsgemisch gaschromatographisch analysiert. Die Ausbeute beträgt 85 % bei 100 % Umsatz.

Nach den oben beschriebenen Verfahren wurden auf analoge Weise folgende Verbindungen hergestellt.

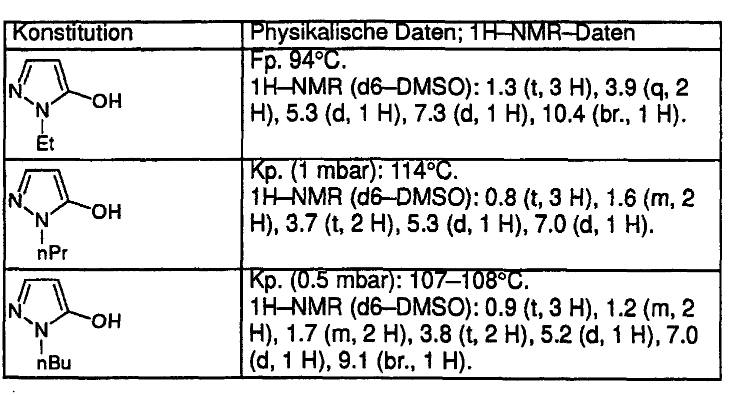

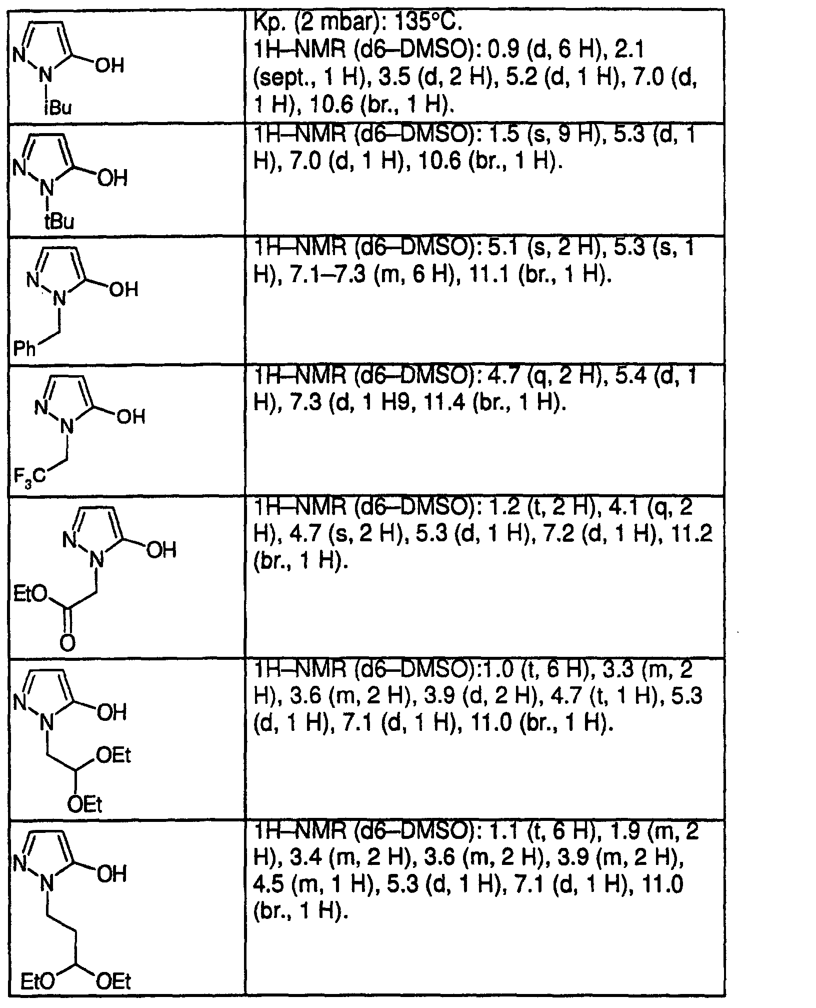

Die nach dem erfindungsgemäßen Verfahren hergestellten 1-substituierten 5- oder 3-Hydroxypyrazole sind wertvolle Vorprodukte zur Herstellung von beispielsweise Pflanzenschutzmitteln wie Herbiziden. Aus der WO 96/26206 bekannte Herbizide sind beispielsweise

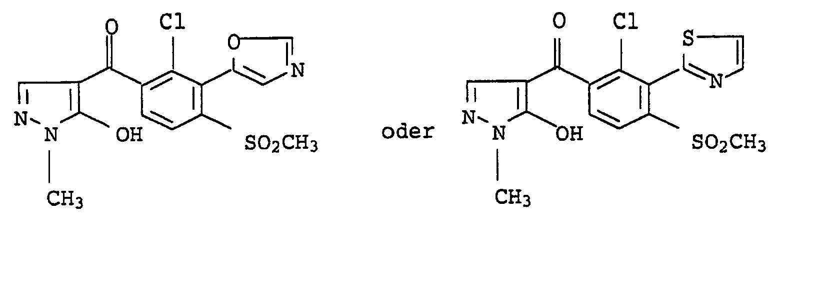

## Patentansprüche

1. Verfahren zur Herstellung von 1-substituierten 5- und/oder 3-Hydroxypyrazolen der Formeln I bzw. II, 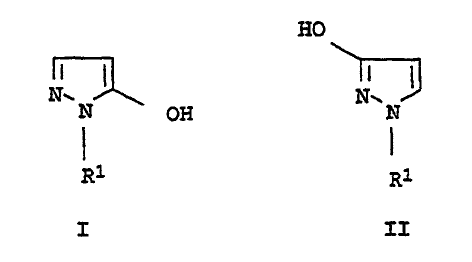 in denen R¹ C₁-C₆-Alkyl, C₂-C₆-Alkenyl, C₂-C₆-Alkinyl, C₃-C₆-Cycloalkyl oder C₁-C₄-Alkoxy bedeutet, wobei diese Gruppen durch Halogen, C₁-C₄-Alkoxy, Phenoxy, C₁-C₆-Alkoxycarbonyl, C₁-C₆-Alkylthiocarbonyl oder ein cyclisches Ringsystem mit 3-14 Ringatomen substituiert sein können, **dadurch gekennzeichnet, daß** man
a) einen Alkylvinylether der allgemeinen Formel V, 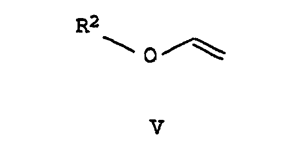 in der R² C₁-C₆-Alkyl oder C₃-C₆-Cycloalkyl bedeutet, mit Phosgen VIa, "Diphosgen" VIb oder "Triphosgen" VIc 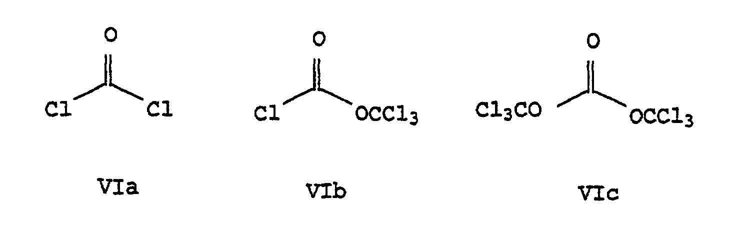 zu Säurechloriden der Formel VII 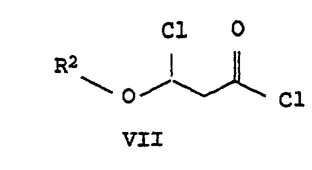 umsetzt,
b) diese durch Eliminierung von Chlorwasserstoff in das entsprechende 3-Alkoxyacrylsäurechlorid der Formel VIII 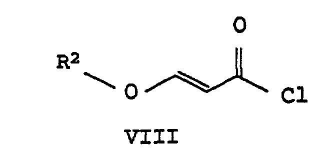 überführt und
c) dieses mit einem Alkohol der Formel IX
R³ ― OH IX
in der R³ C₁-C₆-Alkyl oder C₃-C₆-Cycloalkyl bedeutet, zu dem entsprechenden 3-Alkoxyacrylsäurealkylester der Formel III verestert,
diesen 3-Alkoxyacrylsäurealkylester der Formel III, 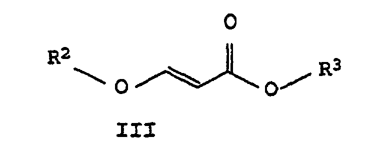 mit einem Hydrazin der Formel IV 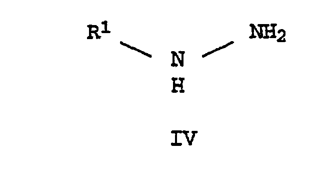 in der R¹ die oben angegebene Bedeutung hat,
d) bei einem pH-Wert von 6-11 zu 5-Hydroxypyrazolen der Formel I umsetzt,
oder
e) bei einem pH-Wert von 11-14 zu 3-Hydroxypyrazolen der Formel II umsetzt.

2. Verfahren nach Anspruch 1, **dadurch gekennzeichnet, daß** man die Umsetzung in Stufe d) bei einer Temperatur von -30°C bis 100°C durchführt.

3. Verfahren nach Anspruch 1, **dadurch gekennzeichnet, daß** man die Umsetzung in Gegenwart einer Base durchführt.

4. Verfahren nach Anspruch 1, **dadurch gekennzeichnet, daß** man als Base Alkalihydroxide, Erdalkalihydroxide oder tert. Amine verwendet.

5. Verfahren nach Anspruch 2, **dadurch gekennzeichnet, daß** man ein Lösungsmittel vorlegt und den 3-Alkoxyacrylsäurealkylester III und das Hydrazin IV gleichzeitig in das Lösungsmittel dosiert.

6. Verfahren nach Anspruch 5, **dadurch gekennzeichnet, daß** man als Lösungsmittel Wasser, Alkohole, Ether oder deren Gemische verwendet.

7. Verfahren nach Anspruch 1, **dadurch gekennzeichnet, daß** man die Umsetzung in Stufe e) bei einer Temperatur von -30°C bis 100°C durchführt.

8. Verfahren nach Anspruch 7, **dadurch gekennzeichnet, daß** man die Umsetzung in Gegenwart einer Base durchführt.

9. Verfahren nach Anspruch 8, **dadurch gekennzeichnet, daß** man als Base Alkalihydroxide, Erdalkalihydroxide, tert. Amine oder deren Gemische verwendet.

10. Verfahren nach Anspruch 1, **dadurch gekennzeichnet, daß** man die Umsetzung in Stufe a) bei einer Temperatur von -78°C bis 100°C durchführt.

11. Verfahren nach Anspruch 1, **dadurch gekennzeichnet, daß** man den Alkylvinylether V mit Phosgen VIa, Diphosgen VIb oder Triphosgen VIc im Molverhältnis 0,1:1 bis 1:1 umsetzt.

12. Verfahren nach Anspruch, 1, **dadurch gekennzeichnet, daß** man die Umsetzung in Stufe b) bei einer Temperatur von 30°C bis 80°C durchführt.

13. Verfahren nach Anspruch 1, **dadurch gekennzeichnet, daß** man die Veresterung in Stufe c) bei einer Temperatur von -20°C bis 80°C durchführt.

## Claims

1. A process for preparing a 1-substituted 5- and/or 3-hydroxypyrazole of the formulae I and II, respectively 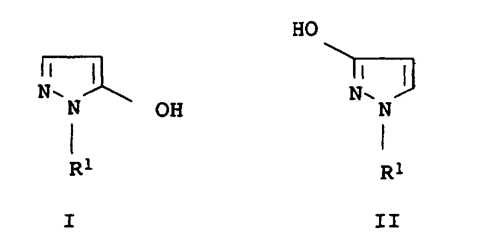 in which R¹ is C₁-C₆-alkyl, C₂-C₆-alkenyl, C₂-C₆-alkynyl, C₃-C₆-cycloalkyl or C₁-C₄-alkoxy, where these groups may be substituted by halogen, C₁-C₄-alkoxy, phenoxy, C₁-C₆-alkoxycarbonyl, C₁-C₆-alkylthiocarbonyl or by a cyclic ring system having 3-14 ring atoms, which comprises reacting
a) an alkyl vinyl ether of the formula V 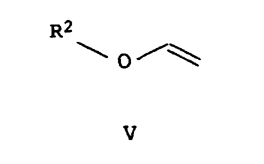 in which R² is C₁-C₆-alkyl or C₃-C₆-cycloalkyl with phosgene VIa, "diphosgene" VIb or "triphosgene" VIc 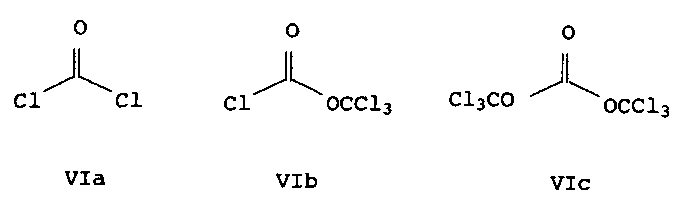 to give an acyl chloride of the formula VII 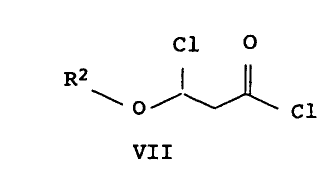
b) converting this by elimination of hydrogen chloride into the corresponding 3-alkoxyacryloyl chloride of the formula VIII 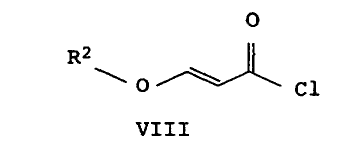 and
c) esterifying this with an alcohol of the formula IX
R³ ― OH IX
in which R³ is C₁-C₆-alkyl or C₃-C₆-cycloalkyl to give the corresponding alkyl 3-alkoxyacrylate of the formula III,
and reacting said alkyl 3-alkoxyacrylate of the formula III 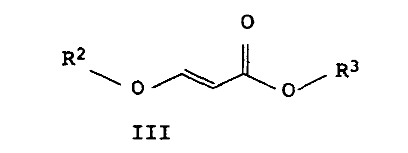 with a hydrazine of the formula IV 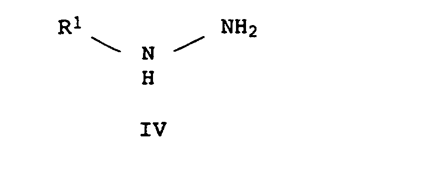 in which R¹ is as defined above
d) at a pH of 6-11 to give 5-hydroxypyrazoles of the formula I
or
e) at a pH of 11-14 to give 3-hydroxypyrazoles of the formula II.

2. A process as claimed in claim 1, wherein the reaction in step d) is carried out at from -30°C to 100°C.

3. A process as claimed in claim 1, wherein the reaction is carried out in the presence of a base.

4. A process as claimed in claim 1, wherein the base used is an alkali metal hydroxide, alkaline earth metal hydroxide or tertiary amine.

5. A process as claimed in claim 2, wherein a solvent is initially charged and the alkyl 3-alkoxyacrylate III and the hydrazine IV are simultaneously metered into the solvent.

6. A process as claimed in claim 5, wherein the solvent used is water, an alcohol, an ether or a mixture of these.

7. A process as claimed in claim 1, wherein the reaction in step e) is carried out at from -30°C to 100°C.

8. A process as claimed in claim 7, wherein the reaction is carried out in the presence of a base.

9. A process as claimed in claim 8, wherein the base used is an alkali metal hydroxide, alkaline earth metal hydroxide, a tertiary amine or a mixture of these.

10. A process as claimed in claim 1, wherein the reaction in step
a) is carried out at from -78°C to 100°C.

11. A process as claimed in claim 1, wherein the alkyl vinyl ether V is reacted with phosgene VIa, diphosgene VIb or triphosgene VIc in a molar ratio of from 0.1:1 to 1:1.

12. A process as claimed in claim 1, wherein the reaction in step b) is carried out at from 30°C to 80°C.

13. A process as claimed in claim 1, wherein the esterification in step c) is carried out at from -20°C to 80°C.

## Revendications

1. Procédé de préparation de 5- et/ou 3-hydroxypyrazoles substitués en position 1, de formule I ou II 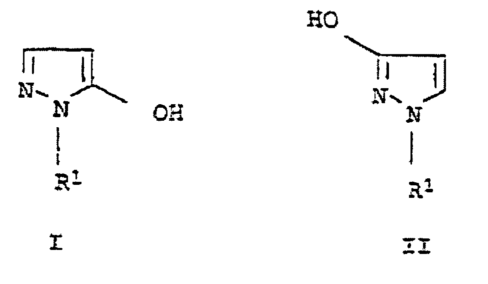 dans lesquelles R¹ représente un groupe alkyle en C₁-C₆, alcényle en C₂-C₆, alcinyle en C₂-C₆, cycloalkyle en C₃-C₆ ou alcoxy en C₁-C₄, ces groupes pouvant être substitués par un atome d'halogène, un groupe alcoxy en C₁-C₄, phénoxy, (alcoxy en C₁-C₆)carbonyle, (alkyle en C₁-C₆)thiocarbonyle en C₁-C₆ ou par un système cyclique de 3 à 14 maillons, **caractérisé en ce que**
a) on fait réagir un alkylvinyléther de formule générale V 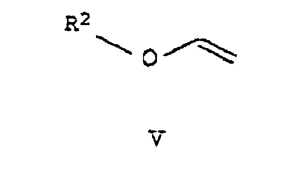 dans laquelle R² représente un groupe alkyle en C₁-C₆ ou cycloalkyle en C₃-C₆, avec du phosgène VIa, du "diphosgène" Vlb ou du "triphosgène" Vlc 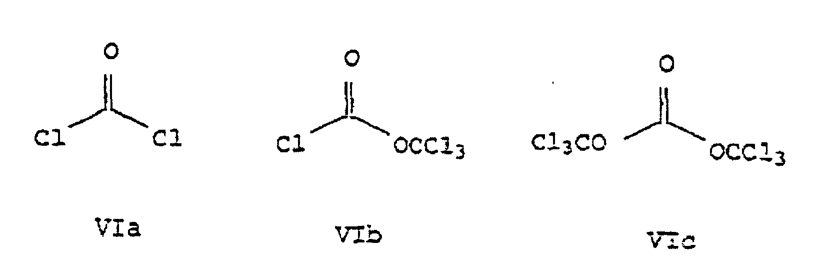 pour obtenir un chlorure d'acide de formule VII 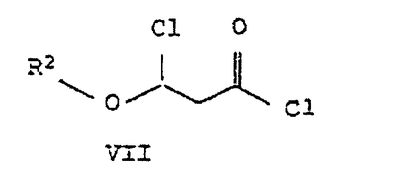
b) on transforme celui-ci par élimination d'acide chlorhydrique en chlorure d'acide 3-alcoxyacrylique correspondant, de formule VIII 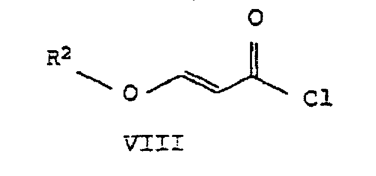 et
c) on estérifie celui-ci avec un alcool de formule IX
R³ ― OH IX
dans laquelle R³ représente un groupe alkyle en C₁-C₆, ou cycloalkyle en C₃-C₆ en l'ester alkylique d'acide 3-alcoxyacrylique correspondant, de formule III,
on fait réagir cet ester alkylique d'acide 3-alcoxyacrylique de formule III 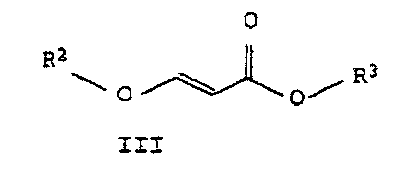 avec une hydrazine de formule IV 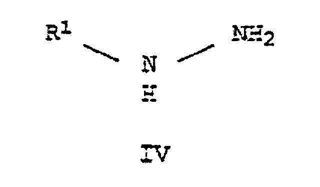 dans laquelle R¹ a la signification mentionnée ci-dessus,
d) à un pH de 6 à 11 pour obtenir des 5-hydroxypyrazoles de formule I
ou
e) à un pH de 11 à 14 pour obtenir des 3-hydroxypyrazoles de formule II.

2. Procédé selon la revendication 1, **caractérisé en ce que** dans l'étape d), on effectue la réaction à une température de -30°C à 100°C.

3. Procédé selon la revendication 1, **caractérisé en ce que** l'on effectue la réaction en présence d'une base.

4. Procédé selon la revendication 1, **caractérisé en ce que** comme base, on utilise des hydroxydes de métaux alcalins, des hydroxydes de métaux alcalino-terreux ou des amines tertiaires.

5. Procédé selon la revendication 2, **caractérisé en ce que** l'on introduit d'abord un solvant et ensuite, on introduit dans ce solvant progressivement et simultanément l'ester alkylique d'acide 3-alcoxyacrylique de formule III et l'hydrazine de formule IV.

6. Procédé selon la revendication 5, **caractérisé en ce que** comme solvant, on utilise de l'eau, des alcools, des éthers ou des mélanges de ceux-ci.

7. Procédé selon la revendication 1, **caractérisé en ce que** dans l'étape e), on effectue la réaction à une température de -30°C à 100°C.

8. Procédé selon la revendication 7, **caractérisé en ce que** l'on effectue la réaction en présence d'une base.

9. Procédé selon la revendication 8, **caractérisé en ce que** comme base, on utilise des hydroxydes de métaux alcalins, des hydroxydes de métaux alcalino-terreux, des amines tertiaires ou des mélanges de ceux-ci.

10. Procédé selon la revendication 1, **caractérisé en ce que** dans l'étape a), on effectue la réaction à une température de -78°C à 100°C.

11. Procédé selon la revendication 1, **caractérisé en ce que** l'on fait réagir l'alkylvinyléther de formule générale V avec du phosgène VIa, du diphosgène Vlb ou du triphosgène VIc, dans un rapport molaire de 0,1:1 à 1:1.

12. Procédé selon la revendication 1, **caractérisé en ce que** dans l'étape b), on effectue la réaction à une température de 30°C à 80°C.

13. Procédé selon la revendication 1, **caractérisé en ce que** dans l'étape c), on effectue l'estérification à une température de -20°C à 80°C.
